# EUROPEAN PATENT APPLICATION

(11) **EP 3 834 724 A2**
(43) Date of publication of application: **16.06.2021**
(21) Application number: 21150771.0
(22) Date of filing: 02.05.2017
(51) Int. Cl.: A61B 5/151, A61B 5/15

(54) **DISPOSABLE PAINLESS LANCET AND LANCING DEVICES FOR USE THEREWITH**

(30) Priority: 02.05.2016 KR 20160053935; 06.07.2016 KR 20160085674; 22.03.2017 KR 20170036096
(62) Divisional of application: 17792887.6
(71) Applicant: Choi, Lim Chel, Busan 49409 (KR); Choi, Jeong Won, Pyeongchang-gun, Gangwon-do 25333 (KR)
(72) Inventor: CHOI, Ming Gi, Busan 46759 (KR); HA, Ho Im, Gangwon-do 25333 (KR); CHOI, Lim Chel, Gangwon-do 25333 (KR); CHOI, Jeong Won, Gangwon-do 25333 (KR)
(74) Representative: Haseltine Lake Kempner LLP

(57) **Abstract**

Provided is a lancing device including a casing, a launch part, and a bumper part wherein the bumper part comprises a needle body holder, a cylindrical bumper and bump spring; and a disposable painless lancet for use with the device. The disposable painless lancet and lancing device according to the present invention are figured such that a bump spring strikes the skin in advance before the needle penetrates the skin, disturbing the skin nerve, and such that a needle of the lancet can be allowed to be inserted only a certain depth of skin, thus the subject does not feel any pain at all.

## Description

### FIELD OF THE INVENTION

The present invention relates to disposable painless lancets and lancing devices for use therewith, which the subject can hardly feel to such an extent that a pain can be neglected. The lancets and the lancing devices of the present invention are configured such that a bump spring strikes a skin in advance before a needle penetrates the skin so as to disturb the skin nerve, and further configured such that, after the needle stabs the skin, the needle can be momentarily released out of the skin due to the restoring force of the bump spring so that a time required for the needle to stay in the skin is very short, and the needle can be adjusted to penetrate deep or shallow by allowing the needle to be inserted only a certain depth of skin.

### BACKGROUND OF THE INVENTION

A small amount of blood can be shed to remove coagulum and metabolic waste matter in oriental medicine. In addition, a small amount of blood can be also sampled to measure blood sugar, blood type, and other blood tests in western medicine. In this case, the subject can feel frightened by the pain of the lancet, i.e., the pain caused by the needle.

A human skin is generally divided into an epidermis (about 0.2 mm), a dermis (about 2 to 3 mm) and a subcutaneous tissue. The subcutaneous tissue has arteries and veins, and in the dermis, fine capillary blood vessels are distributed like a network. In order to sample blood, it is necessary to penetrate the needle up to the capillary blood vessels, that is, just before the subcutaneous tissue. Conventionally, as the needle stabs the skin, the needle can pass through the subcutaneous tissue and penetrate excessively too deeply, resulting in a severe pain. In order to solve such a problem, lancets having various lengths are commercially available, and the user has an inconvenience of using a lancet suitable for him as needed.

Korean Patent No. 10-1360939 discloses a lancet which does not penetrate the skin through a certain depth or more but merely draws blood, wherein the lancet has double bumps (multi-bumps) each having a cross-sectional area wider than that of a middle of the needle, thereby preventing the needle from being excessively deeply inserted. In the patent, the lancet is intended to reduce a length of the needle slightly so that the needle can penetrate slightly into the skin to reduce pain. Since a thickness of a human skin varies depending on a skin condition of a person, there is a disadvantage that a penetrating depth of the needle cannot be adjusted. In addition, even though the needle of the lancet is as short as possible, if the needle is directly inserted into the skin, the subject feels pain, so that a true painless effect cannot be obtained.

A complicated device using a projectile, a trigger, and a laser is known to prevent the needle from reaching a certain depth or more of the skin. However, since a disposable lancet is discarded after being used once, its price should be low and its structure should be simple.

Also, even if the lancet is disposable, there is always a risk that secondary infections such as AIDS and hepatitis can be infected unless special care is taken after use.

Prior arts related to a device for sampling blood include the following. For example, Korean Patent No. 10-0932946 discloses a device for securing a straight direction when a lancing needle penetrates the skin, but the device is not related to a function of eliminating a pain.

Korean Patent No. 10-0912202 discloses a lancet integrated cap and a painless lancet. The patent describes caps **210, 310, 510** for accommodating a lancet body **140, 220, 320, 520** as the striking means, similar to the present invention. However, since the caps as the striking means must be spaced apart from the skin to strike the skin, it is difficult for the user to maintain a proper distance. For example, if the distance between the skin and the cap is too far compared to the proper distance, the needle will not penetrate the skin. Conversely, if the distance between the skin and the cap is too close compared to the proper distance, there will be no striking effect. Since the striking strength and the depth of penetration of the needle vary according to the user, a consistency of a painless effect cannot be maintained. Therefore, there is a problem that a perfect painless effect cannot be expected in this device. In addition, since a lancet body and the cap are integrated, both the lancet body and the cap should be disposed of after use. Therefore, there is a problem in that the manufacturing cost is increased as well as a waste of resources.

### DETAILED DESCRIPTION OF THE INVENTION

### PROBLEMS TO BE SOLVED

As a result of researching to solve the above-mentioned problems, the present inventors have found: that the subject cannot feel pain when the needle is stuck by providing a coil-type bump spring embedded or fixed in the needle body so as to surround the needle and to protrude longer than the needle, since the bump spring strikes around the skin instantly before the needle of the lancet pierces the skin, thereby disturbing the skin nerve and allowing the needle to stay a very short time in the skin; that the problem of secondary infection due to careless handling after use can be solved, since the bump spring surrounds and protects the needle; and that it is possible to prevent the needle from excessively and deeply penetrating to below the subcutaneous tissue, since the needle penetrates the skin only as much as the length of the needle protruding from the bump spring when the bump spring is compressed by impact. The present invention has been completed based on these finding.

Therefore, an object of the present invention is to provide a simple and inexpensive disposable painless lancet and a device for use therewith, wherein the bump spring is provided with the needle body to disturb the skin nerve, the needle is allowed to penetrate only a certain depth of the skin, and the needle can be adjusted to pierce the skin deep or shallow, so that the subject can hardly feel the pain and the blood on the needle does not come into contact with the outside after use.

### SOLUTION TO THE PROBLEM

An aspect of the first embodiment of the present invention provides a painless lancing device.

The painless lancing device comprises a casing which includes a cylindrical front cap, a sleeve connected to the cylindrical front cap at a lower end and a rear cap connected to an upper end of the sleeve, a launch part disposed in the casing configured to launch a cylindrical bumper and a needle body to strike a skin. The bumper part is disposed in the casing and connected to the launch part by a coupling shaft.

Further, the bumper part includes a needle body holder which has a holder flange disposed at an upper portion thereof, a first rectangular hole disposed below the holder flange so that a stop bar moves up and down, and a needle body insertion hole disposed at a lower portion of the bumper part to insert a lancet. The cylindrical bumper is configured to receive the needle body holder at an upper end thereof, and further includes a second rectangular hole disposed along a longitudinal direction to insert and remove the lancet, a needle access hole disposed at a lower end of the cylindrical bumper for a needle, a needle spring to move in and out therethrough, and a stop bar insertion port disposed at the upper end to insert the stop bar. Further, the lancing device includes a bump spring surrounding a cylindrical body and disposed between a lower portion of the holder flange of the needle body holder and an upper portion of the cylindrical bumper.

Another aspect of the first embodiment of the present invention provides the painless lancing device, wherein one side of the needle body insertion hole is configured to allow insertion and removal of the needle body from said one side, and the needle body is disposed to align and the upper and lower surfaces of the needle body insertion hole are aligned with the upper and lower surfaces of the flat needle body.

Still another aspect of the first embodiment of the present invention provides the painless lancing device, wherein the cylindrical body has a lower end configured to allow the needle of the lancet and the needle spring to move in and out, wherein a ring-shaped protrusion is disposed at an inside surface of an upper end of the holder flange so that the ring-shaped protrusion is engaged with a circular-shaped coupling groove disposed along a lower outer peripheral surface of the coupling shaft, and wherein the holder flange is disposed on its outer circumferential surface thereof for screw coupling with a spiral disposed on an inside surface of a depth adjusting member.

Still another aspect of the first embodiment of the present invention provides the painless lancing device, wherein the upper and lower surfaces of the needle body insertion holes have needle body fixing protrusions for fixing the needle body.

Still another aspect of the first embodiment of the present invention provides the painless lancing device, wherein the lancet further includes a rectangular needle body having protrusions on both sides thereof, a needle fixing protrusion for inserting and fixing the needle springs on the protrusion, a needle inserted into the needle body through the needle fixing protrusion, and fixing grooves to be fixed on the needle body fixing protrusions provided on both inner walls of the needle body insertion hole.

Still another aspect of the first embodiment of the present invention provides the painless lancing device, which further includes a depth adjusting member configured to adjust a depth of skin penetration of the needle and having an open upper end and an open lower end, the depth adjusting member including a spiral disposed in an inner surface thereof for engaging with a screw thread disposed on an outer circumferential surface of the holder flange of the needle body holder, and an adjusting member flange disposed at a lower end of the depth adjusting member.

Still another aspect of the first embodiment of the present invention provides the painless lancing device, wherein the bumper part is connected to the coupling shaft by engaging a ring-shaped protrusion disposed at an inside surface of the holder flange with a circular coupling groove disposed along a lower portion of the coupling shaft, the coupling shaft having a coupling shaft flange for inserting a return spring therein.

An aspect of the second embodiment of the present invention provides a disposable painless lancet including a needle body, a needle embedded at the center of one end of the needle body, and a bump spring embedded or fixed at said one end of the needle body so as to surround the needle and extend beyond the needle.

An aspect of the second embodiment of the present invention provides the disposable painless lancet, wherein the needle body has an elongated spiral part disposed at one end thereof; wherein one end of the needle is embedded in the center of the end of the spiral part; wherein a spring body in the form of a nut having a spiral disposed therein so as to be screwed with the elongated spiral part is included; and wherein one end of the bump spring is embedded or fixed to the spring body.

Still another aspect of the second embodiment of the present invention provides the disposable painless lancet, wherein a silicone or elastic member is used instead of the bump spring.

Still another aspect of the second embodiment of the present invention provides a painless lancing device including: a casing including a cylindrical front cap, a sleeve having one side connected to the front cap and the other side connected to a rear cap which is connected with the sleeve; a launch part for launching a needle body disposed inside a cylindrical body; and a needle body holder for holding and fixing the needle body. The cylindrical front cap has a gradually tapering shape from a top end to a bottom end, and includes a circular engagement protrusion disposed on the inside of the front cap to limit the downward movement of the lower surface of the cylindrical body and a guide hole provided from the engagement protrusion to the bottom end of the front cap to guide the needle body and a bump spring in a predetermined direction. The needle body holder includes a ring-shaped protrusion disposed inside the needle body holder so as to be engaged with a coupling groove of the coupling shaft, and a needle body holder is open at its bottom portion and has one incision groove cut from the lower portion to a middle portion thereof.

### EFFECTS OF THE INVENTION

The painless lancet and the lancing device to use therewith according to the present invention can control the needle to be inserted only to a certain depth of the skin without changing various sizes of the needle so that the needle does not penetrate below the subcutaneous tissues. The needle is instantly released out of the skin due to the restoring force of the bump spring after the needle has pierced the skin, and the time required for the needle to stay in the skin is shortened, and thus the subject does not feel any pain at all.

### BRIEF DESCRIPTION OF THE DRAWINGS

FIG. **1** is a perspective view of the lancing device according to a first embodiment of the present invention.
FIG. **2** is a perspective view of the lancing device according to the first embodiment of the present invention showing a state in which a front cap, a sleeve, and a rear cap, which form an external appearance, are disassembled.
FIG. **3** is a longitudinal sectional view of the lancing device according to the first embodiment of the present invention cut out at an angle of 90 degrees.
FIG. **4** is a longitudinal sectional view of the lancing device according to the first embodiment of the present invention.
FIG. **5** is a longitudinal sectional view of the lancing device according to the first embodiment of the present invention in which a front cap, a sleeve, and a rear cap are omitted.
FIG. **6** is a drawing showing an internal structure of the lancing device according to the first embodiment of the present invention.
FIG. **7** is a drawing showing an operating state of a launch part.
FIG. **8** is a cross-sectional view of a bumper part and a coupling shaft of the lancing device according to the first embodiment of the present invention, in which the bumper part is coupled with the coupling shaft.
FIG. **9** is a drawing showing a state in which a needle body holder and a depth adjusting member of the bumper part of the lancing device according to the first embodiment of the present invention are disassembled and the needle body holder and the coupling shaft are engaged.
FIG. **10** is an enlarged perspective view of the bumper part of the lancing device according to the first embodiment of the present invention.
FIG. **11** is an exploded perspective view of the bumper part of the lancing device according to the first embodiment of the present invention.
FIG. **12** is a drawing showing a state in which the depth adjusting member of the lancing device according to the first embodiment of the present invention is engaged with the needle body holder, and the needle body is inserted into a needle body hole or disassembled therefrom.
FIG. **13** is an enlarged longitudinal sectional view of the bumper part for showing an operating state of the bumper part of the lancing device according to the first embodiment of the present invention, in which FIG. **13** (a) illustrates a state of the lancet before launching, and FIG. **13** (b) illustrates a state in which the lancet penetrates the skin immediately after the lancet is launched.
FIG. **14** is a perspective view of a disposable painless lancet according to a second embodiment of the present invention, in which FIG. **14** (a) illustrates a state before use in which the bump spring is expanded, and FIG. **14** (b) illustrates a state in which the needle penetrates the skin and the bump spring is compressed.
FIG. **15** is another perspective view of a disposable painless lancet according to the second embodiment of the present invention.
FIG. **16** is a longitudinal sectional view of the disposable painless lancet according to the second embodiment of the present invention.
FIG. **17** is another perspective view of the disposable painless lancet according to the second embodiment of the present invention, in which the lancet is disassembled.
FIG. **18** is another perspective view of the disposable painless lancet according to the second embodiment of the present invention, in which a silicone or elastic member is used in place of the bump spring, in which FIG. **18** (a) illustrates a state before use in which the silicone or elastic member is expanded, and FIG. **18** (b) illustrates a state in which the needle penetrates the skin and the silicone or elastic member is compressed.
FIG. **19** is another perspective view of the lancing device according to the second embodiment of the present invention illustrating a state in which a front cap, a sleeve, and a rear cap, which form an external appearance, are disassembled.
FIG. **20** is another longitudinal sectional view of the lancing device according to the second embodiment of the present invention.
FIG. **21** is a drawing showing an internal structure of the lancing device according to the second embodiment of the present invention.
FIG. **22** is a cross-sectional view of a holder part and a coupling shaft of the lancing device according to the second embodiment of the present invention, in which the holder part is coupled with the coupling shaft, and the lancet is mounted to the device.
FIG. **23** is a drawing illustrating a structure in which the holder part is coupled with the coupling shaft, and the lancet is mounted to the device in accordance with the second embodiment of the present invention.
FIG. **24** is a drawing illustrating a state in which the rectangular part, the coupling shaft and the holder part of the lancing device according to a second embodiment of the present invention are assembled.
FIG. **25** is a drawing illustrating a state in which the needle body of FIG. **24** is disassembled.
FIG. **26** is a drawing illustrating the operation states of the bump spring and the needle of the lancing device according to the second embodiment of the present invention, in which FIG. 26(a) illustrates a state before launching and FIG. 26(b) illustrates a state after launching.

### THE BEST MODE FOR CARRYING OUT THE INVENTION

Hereinafter, the present invention will be described in more detail with reference to the accompanying drawings. However, the contents shown in the detailed description and in the drawings do not limit the present invention.

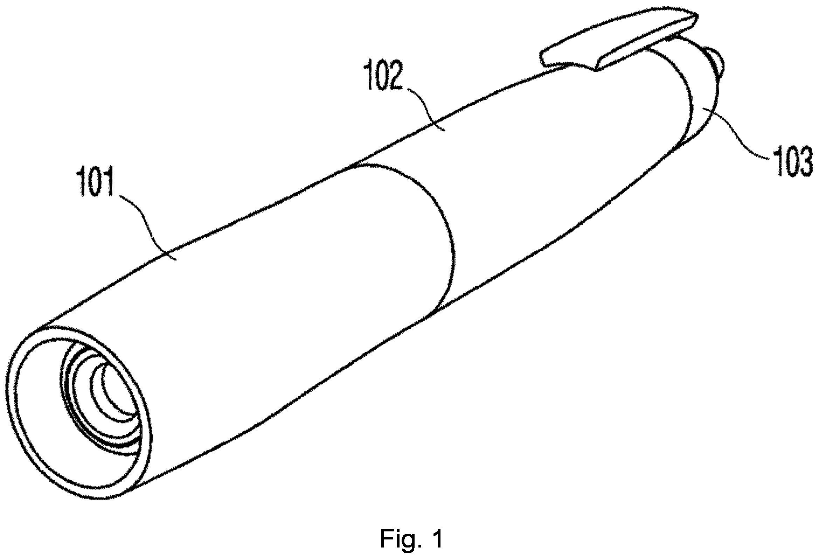

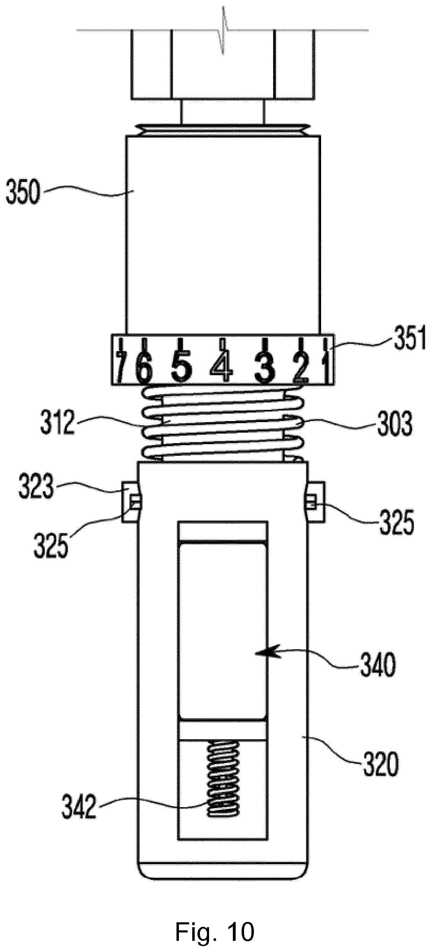

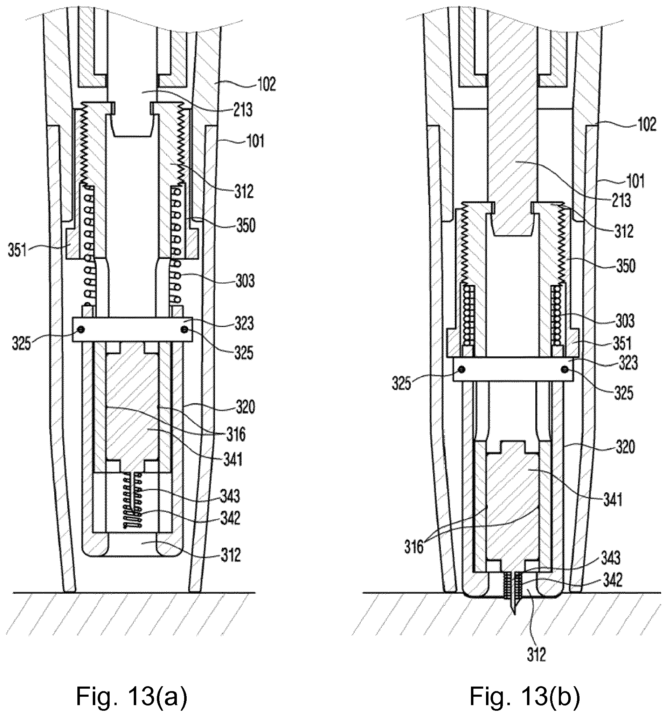

FIGS. **1-13** are views of the lancing device according to a first embodiment of the present invention.

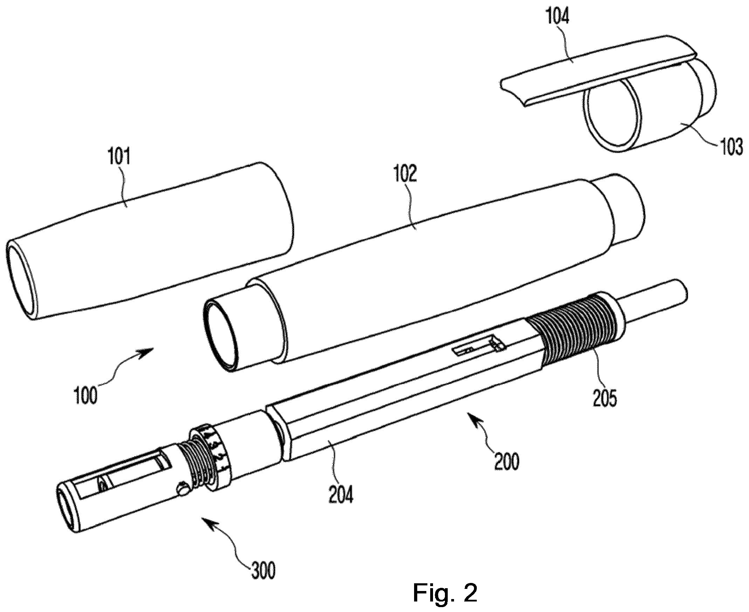

As shown in FIGS. **1** and **2****,** the lancing device according to the first embodiment of the present invention includes: a casing **100** that forms an outer appearance of the device; and a launch part **200** for launching a bumper part **300** having a bumper **320** and a needle body **341** to strike the skin, in which the bumper part **300** has a needle body holder **310** including the bumper **320** that primarily strikes the skin and a needle spring **342** that strikes the skin secondarily.

When the bumper part **300** is launched, the bumper part **300** first strikes the skin by the inertial force at the time of launching, and then continues to press the skin while being in contact with the skin by the compressive force of a bump spring **303.** At this time, the bump spring **303** is compressed due to the inertial force of the bumper **320,** which is momentarily maintained, and the inertial force of the needle body holder **310** including the lancet. A stop bar **323** seated on an upper end of the bumper **320** then moves up to a tip of an adjusting member flange **351** to be properly adjusted along a rectangular hole **313** of a cylindrical body **312,** and the needle spring **342** of the needle body **341** strikes the skin. Immediately thereafter, the needle **343** penetrates into the subcutaneous tissue of the skin, and then the entire bumper part **300**,- that is, the bumper **320,** the needle body holder **310,** a depth adjusting member **350,** the stop bar **323,** a cotter pin **325,** the needle body **341,** and the needle spring **342** are simultaneously returned to the initial position before the launch by a bumper return spring **214.** At this time, a penetration depth of the needle **343** can be controlled by the vertical movement of the stop bar **323** only to the extent of the adjustment position set by the adjusting member flange **351.**

FIG. **1** is a perspective view of a lancing device according to the first embodiment of the present invention, showing the casing **100.** As shown in FIGS. **1** and **2****,** the casing **100** includes a cylindrical front cap **101,** a sleeve **102** having one side coupled to the front cap **101** and the other side coupled to a rear cap **103,** and the rear cap **103** coupled to the sleeve **102.** The front cap **101,** the sleeve **102** and the rear cap **103** are preferably screw-coupled together. The rear cap **103** may be provided with a clip **104** for convenient carrying.

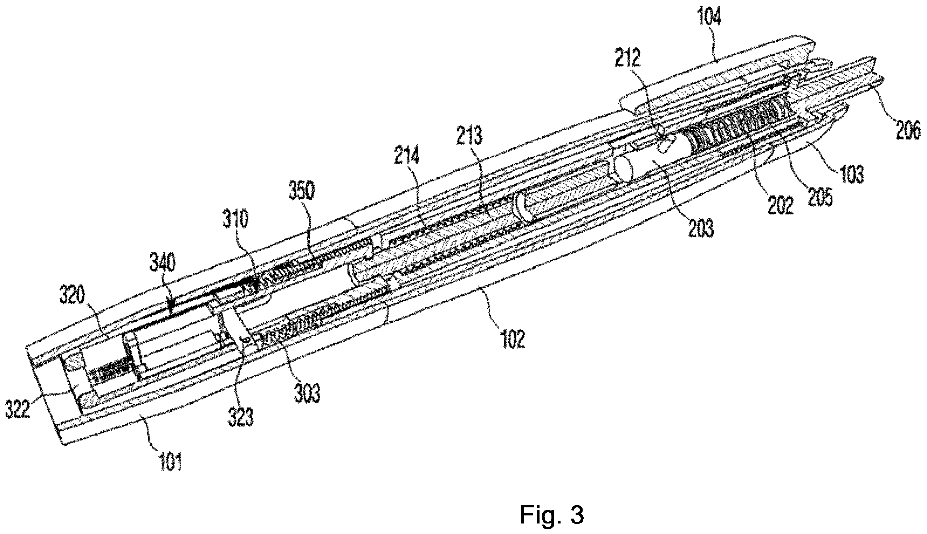

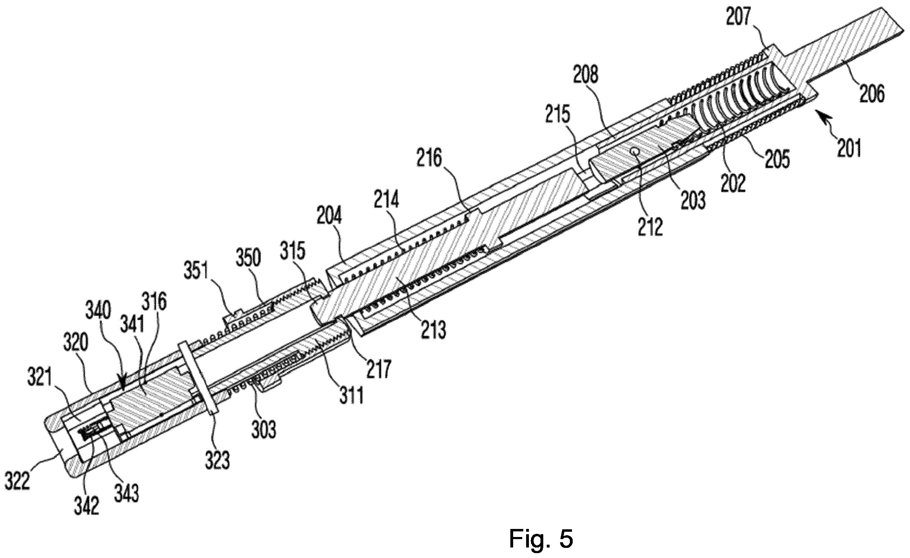

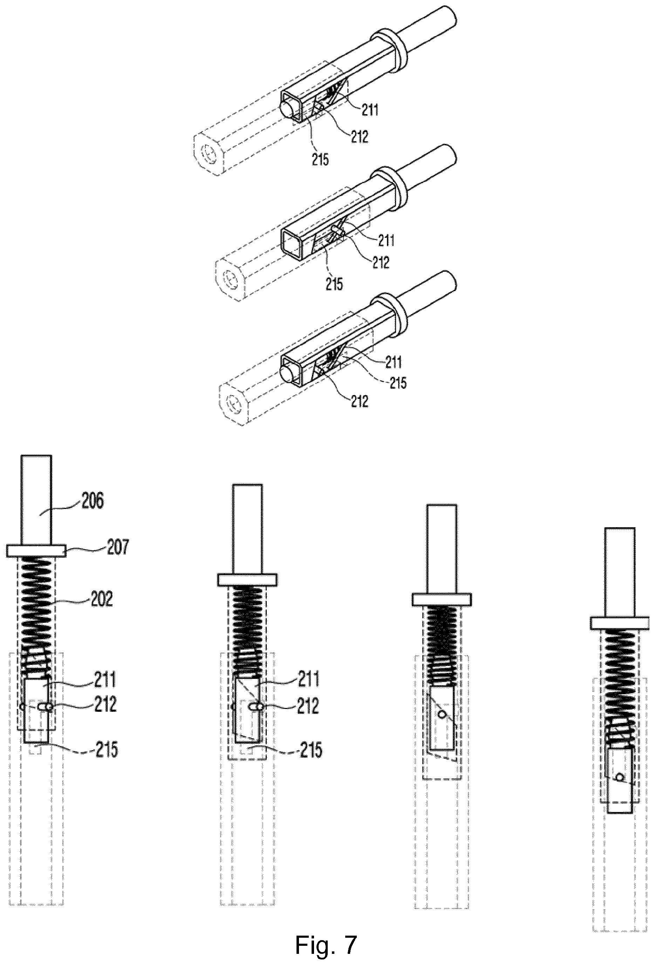

As shown in FIGS. **3-7****,** the launch part **200** includes a launch body **201,** a launch spring **202,** a launch pestle **203,** a square tube **204** and a launch body return spring **205.** As shown in detail in FIGS. **5-7****,** the launch body **201** integrally comprises a push member **206,** a launch flange **207** formed at the tip of the push member **206,** and a hollow tetrahedron **208** which is open at one side to accommodate the launch pestle **203** and the launch spring **202.** As shown in detail in FIG. **7****,** in a lower part of the hollow tetrahedron **208,** there are formed square holes **211** whose upper sides are sloped on one surface and the opposite surface, respectively. The upper sides of the two square holes **211** are opposite to each other (i.e., '\' or '/'). A launch spring **210** is inserted into one side of the launch pestle **203** and a trigger pin **212** is coupled through a middle part of the launch pestle **203.**

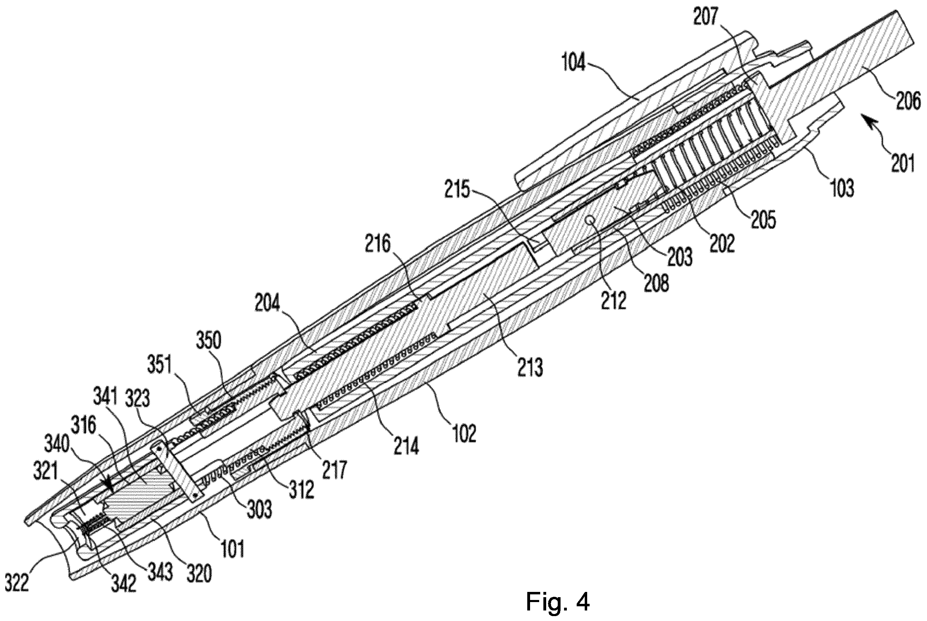

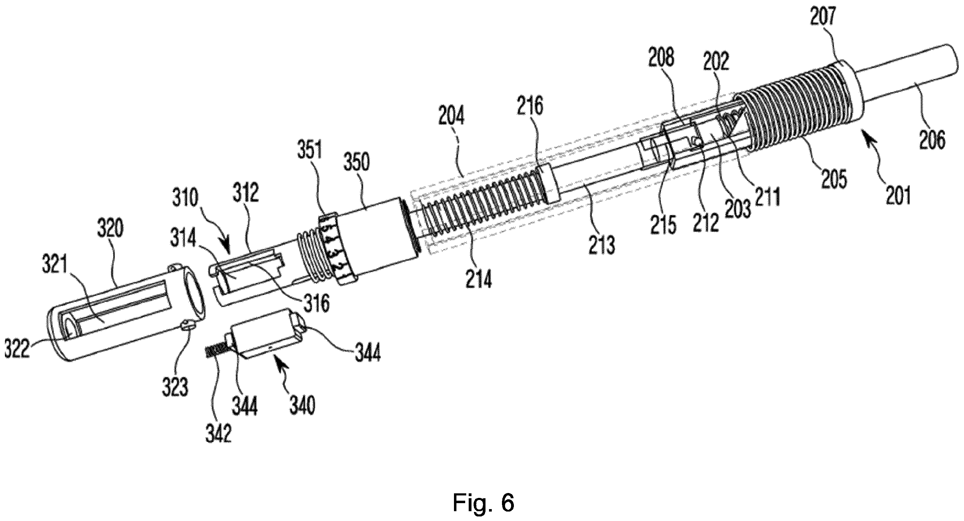

As shown in detail in FIGS. **4-6****,** a rectangular hole (not shown) is formed in the upper part of the square tube **204** to insert the hollow tetrahedron **208** of the launch body **201,** and a circular hole (not shown) is formed in the lower part of the square tube **204** to insert a columnar coupling shaft **213.** As shown in detail in FIGS. **6** and **7****,** the square tube **204** is configured to receive the coupling shaft **213,** the bumper return spring **214** and half of the tetrahedron **208** of the launch body **201.** Two '┐'-shaped slits **215** are formed on one side and the opposite side of the square tube **204,** respectively, and the two slits **215** are formed asymmetrically. That is, one of the two transverse slits **215** has a '┐' shape and the other has a T' shape. The slits **215** are fitted with a trigger pin **212** to move.

A launch body return spring **205** for returning the launch body **201** is interposed between the upper end of the square tube **204** and the launch flange **207.**

Referring to FIG. **7****,** the operation of the launch part **200** will be described as follows.

Before launching, the trigger pin **212** is positioned at the lower side of the square hole **211** and in the transverse slits of the '┐'-shaped slits **215.** The push member **206** is pressed by about 10 mm to launch the bumper **320** and the needle holder **302,** then the launch spring **210** connected to the launch pestle **203** in the hollow tetrahedron **208** is compressed. The trigger pin **212** passing through the launch pestle **203** connected to the launch spring **210** is slid along the upper side of the rectangular hole **211** and is "twisted", and then is pushed along the transverse slits of the '┐'-shaped slits **215** and momentarily falls below the longitudinal slits of the '┐'-shaped slits **215,** whereby the launch pestle **203** can be launched. When the launch pestle **203** is lowered to the end of the transverse slits of the '┐'-shaped slits, a launching force is transmitted to the bumper part **300** through the coupling shaft **213,** so that the bumper **320** strikes the skin. When the push member **206** is pushed again, the launch body **201** and the launch pestle **203** return to the transverse slits of the '┐'-shaped slits **215** and return to the standing state.

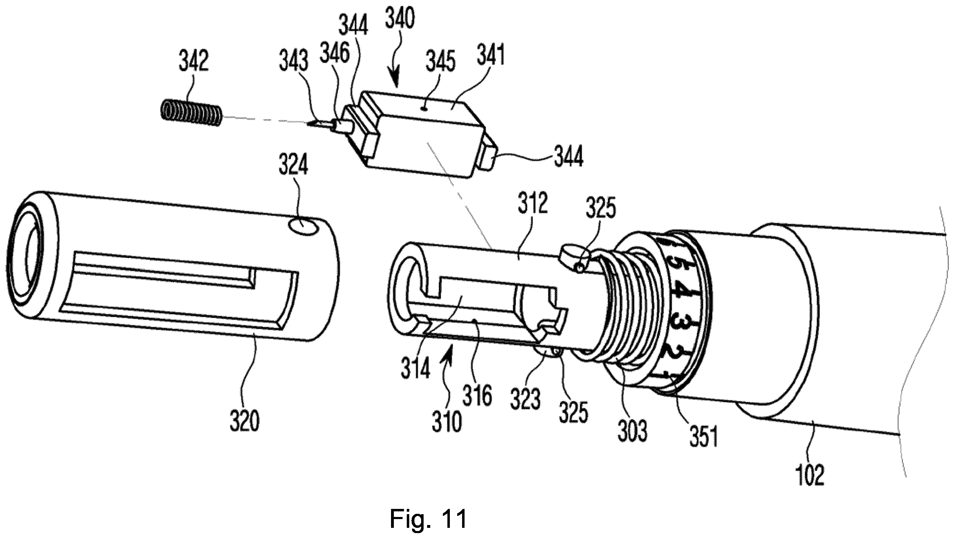

As shown in FIGS. **6** and **11****,** the bumper part **300** mainly includes the needle body holder **310,** the bumper **320,** and the bump spring **303.**

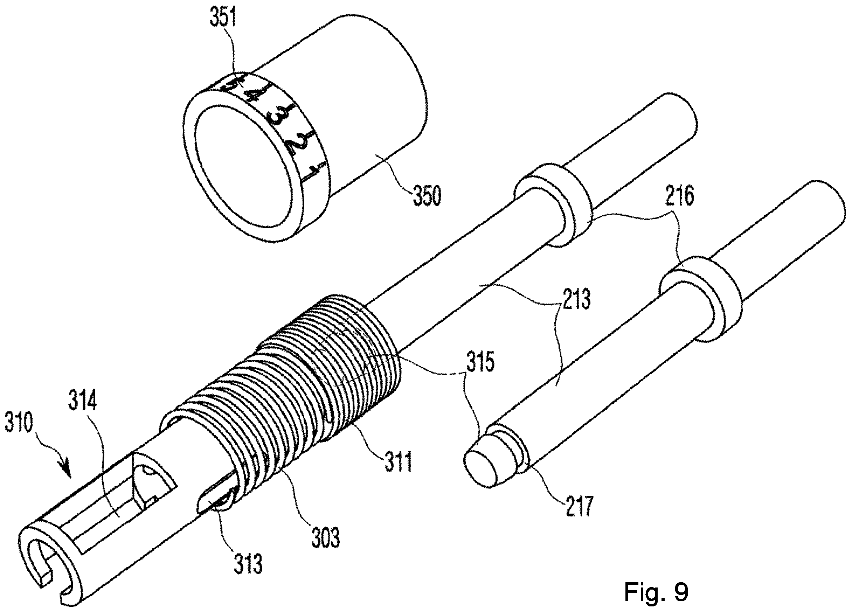

As shown in detail in FIG. **9****,** the needle body holder **310** includes a holder flange **311** formed at its upper portion and a cylindrical body **312** formed under the holder flange **311.** A rectangular hole **313** is formed in the cylindrical body **312** so that the stop bar **323** moves up and down. A needle body insertion hole **314** for inserting a lancet **340** is formed in the lower portion of the cylindrical body **312.**

As shown in FIG. **11****,** one side of the needle body insertion hole **314** is opened to allow the needle body **341** to be inserted and removed laterally, and the two inner walls of the needle body insertion hole **314** are configured to be flat to engage with the two lateral surfaces of the needle body **341.** The lower end of the cylindrical body **312** is opened to allow the needle **343** of the lancet **340** and the needle spring **342** to move in and out. A needle body fixing protrusion **316** fixed to the fixing groove **345** of the needle body **341** is formed on the inner walls of the needle body insertion hole **314.** As shown in FIG. **9****,** a ring-shaped protrusion **315** is formed on the upper end of the holder flange **311** toward the inside of the holder flange **311** so as to be coupled to a circular-shaped coupling groove **217** formed along the outer peripheral surface of the lower end of the coupling shaft **213.** A screw thread for engaging with a spiral formed on the inner side of the depth adjusting member **350** is formed on the outer peripheral surface of the holder flange **311.**

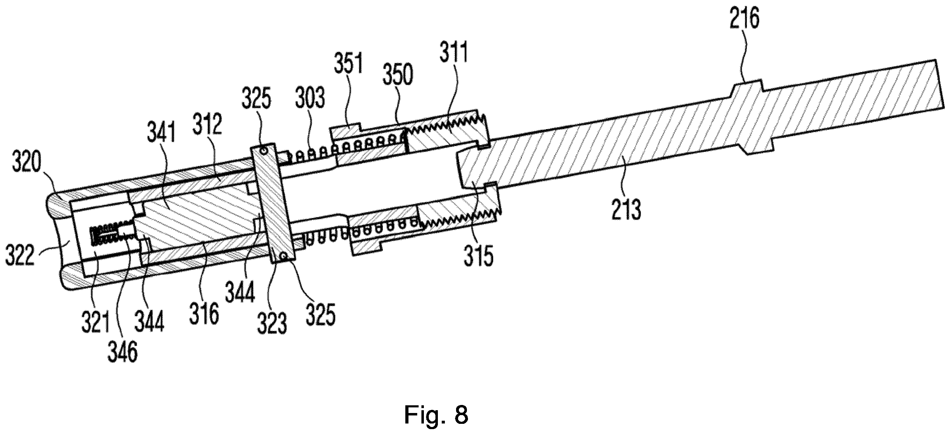

As shown in FIGS. **8** and **9****,** the coupling shaft **213** has a columnar shape and has a coupling shaft flange **216** for inserting and fixing the return spring **214.** The coupling groove **217** is formed at the lower end of the coupling shaft flange **216** so as to be engaged with the ring-shaped protrusion **315** formed toward the inside of the holder flange **311.**

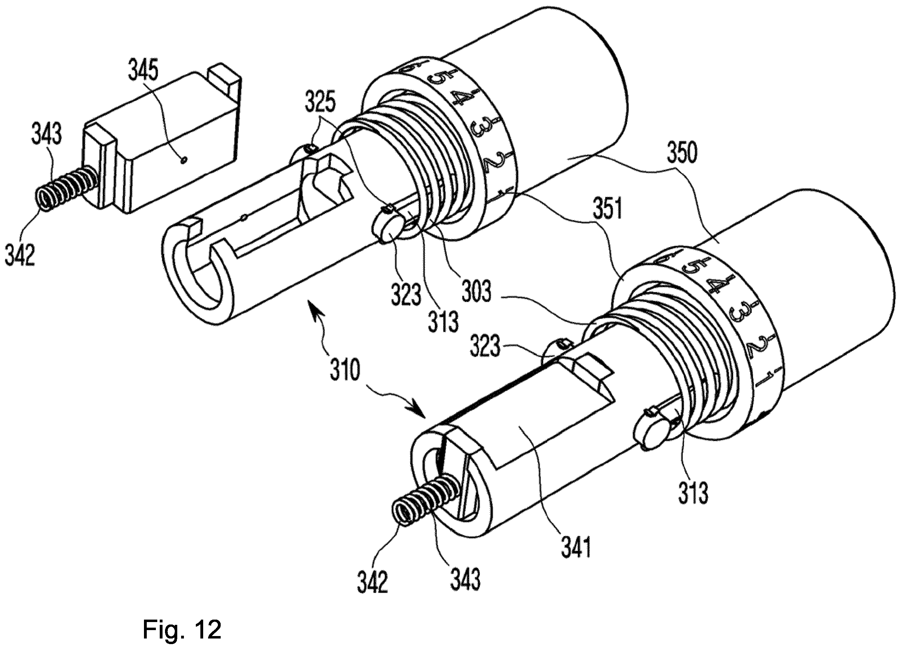

As shown in FIGS. **11** and **12****,** a needle body fixing protrusion **316** for fixing the needle body **341** is formed on the inner walls of the needle body insertion hole **314.** The needle body fixing protrusion **316** is preferably in the form of a bead of a metal or plastic material.

As shown in detail in FIGS. **6** and **11****,** the bumper **320** is cylindrical, and the upper part of the bumper **320** is open to receive the needle body holder **310** and has a rectangular hole **321** for inserting and removing the lancet **340** in the longitudinal direction. A needle access hole **322** is formed at the lower end of the bumper **320** to allow the needle **343** and the needle spring **342** to move in and out. A stop bar insertion port **324** is formed in the upper end of the bumper **320** to insert the stop bar **323.** As shown in detail in FIGS. **8-11****,** the stop bar **323** is inserted through the rectangular hole **313** and the stop bar insertion port **324** of the needle body holder **310.** The cotter pins **325** are fitted at both ends of the stop bar **323** so that the stop bar **323** is not separated from the bumper **320.**

As shown in detail in FIGS. **5****,** **8****,** **10** and **13****,** the bump spring **303** is inserted between the lower surface of the holder flange **311** of the needle body holder **310** and the upper surface of the bumper **320** while surrounding the cylindrical body **312.**

As shown in detail in FIGS. **11-13****,** the lancet **340** inserted into the lancing device according to the first embodiment of the present invention includes the needle body **341,** the needle spring **342,** and the needle **343.** The needle body **341** has protrusions **344** formed at both ends along the longitudinal direction of the needle body **341** and is configured to be inserted in conformity with the shape of the needle body insertion hole **314.** The needle **343** is embedded in the needle body **341.** Fixing grooves **345** are formed on both sides of the needle body **341** so as to be fastened to the needle body fixing protrusions **316** provided on the inner wall of both sides of the needle body insertion hole **314** of the needle body holder **310.**

The lancet **340** according to the first embodiment of the present invention is disposable and the needle spring **342** completely surrounds the needle **343** to prevent secondary infections such as hepatitis and AIDS caused by careless handling.

The lancing device according to the first embodiment of the present invention may be provided with a depth adjusting member **350** to adjust the needle penetration depth since people's skin thickness is different. As shown in detail in FIGS. **4****,** **5****,** and **13****,** the depth adjusting member **350** is cylindrical and is open at its upper and lower ends. A spiral for coupling with a screw formed on the holder flange **311** of the needle body holder **310** is formed on the upper inner circumferential surface of the depth adjusting member **350** and an adjusting member flange **351** is formed at a lower end thereof.

As shown in detail in FIGS. **3** and **4****,** the adjusting member flange **351** is engaged with the screw formed in the holder flange **311** at the top of the cylindrical body **312** of the needle body holder **310,** and the penetration depth of the needle **343** can be adjusted by turning the depth adjusting member **350** to the left or right. When the adjusting member flange **351** is turned to the right, the needle **343** penetrates deeper into the skin. At this time, the stop bar **323** moves along the rectangular hole **313** and the movement distance becomes longer, so that the skin penetrates deeper into the skin. On the contrary, when the adjusting member flange **351** is turned to the left, the needle **343** penetrates the skin more shallowly. At this time, the stop bar **323** moves along the rectangular hole **313** and the movement distance becomes shorter, so that the skin penetrates the skin more shallowly.

Referring to FIGS. **3-5****,** **8****,** **11** and **13**, the operation of the bumper part **300** of the lancing device according to the first embodiment of the present invention will be described below.

When the push member **206** of the launch part **200** is pushed to trigger the launch pestle **203** to strike the coupling shaft **213,** a force is transmitted to the needle body holder **310** through the ring-shaped protrusion **315** formed toward the inner side of the coupling groove **217** of the coupling shaft **213** and the holder flange **311** coupled to the coupling groove **217.** At this time, the bump spring **303** should be strong enough to transmit the launching force to the bumper **320.** The lower end surface of the bumper **320** strikes the skin due to the launching force, then the bump spring **303** is compressed and the needle spring **342** of the needle body **341** inserted into the needle body holder **310** strikes the skin. Immediately thereafter, the needle **343** penetrates the subcutaneous tissue of the skin, and then the entire bumper part **300,** that is, the bumper **320,** the needle spring **342** and the needle **343** are simultaneously returned by the return spring **214.** While the bumper **320** is striking the skin and contacting the skin, the needle spring **342** inserted into the needle body holder **310** strikes the skin, and then the needle **343** penetrates the skin immediately when the needle spring **342** is in contact with the skin. At the same time, the stop bar **323** rises along the rectangular hole **313** of the needle body holder **310** and stops to the lower end of the adjusting member flange **351.** This point is the limit that the needle **343** penetrates the skin. At this time, the depth of penetration of the needle **343** is adjusted by causing the stop bar **323** to perform a vertical movement only to the end of the screw adjusting position of the adjusting member flange **351.** After the bumper **320** strikes the skin, the needle spring **342** strikes the skin again in contact with the skin. Further, after the needle **343** pierces the skin while the bumper **320** and the needle spring **342** are in contact with the skin, the bumper **320,** the needle spring **342,** and the needle **343** are simultaneously released from the skin by the bumper return spring **214.** The needle spring **342** extends a little longer than the needle **343** and is inserted or fixed to the needle fixing protrusion **346** formed on the protrusion **344** while surrounding the needle **343.**

As described above, since the bumper **320** first strikes the skin, and then the needle spring **342** strikes the skin secondarily with a time difference of about 0.1 second, the needle **343** pierces the skin almost at the same time, and the subject will not feel any pain at all.

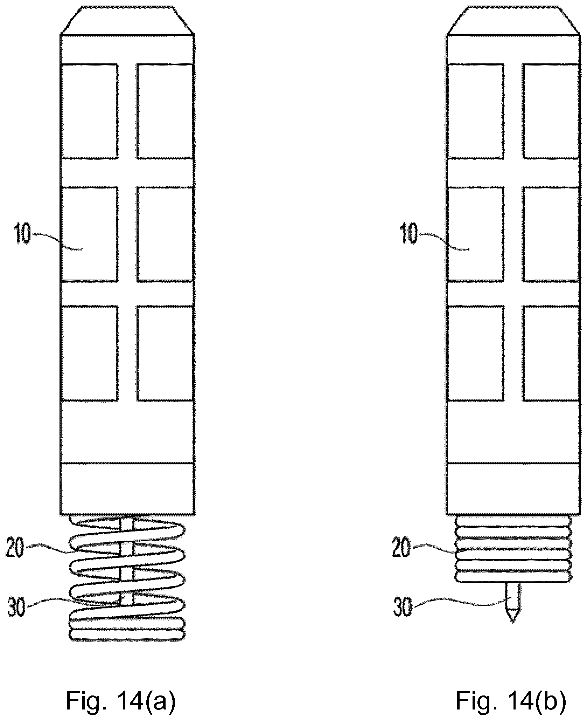

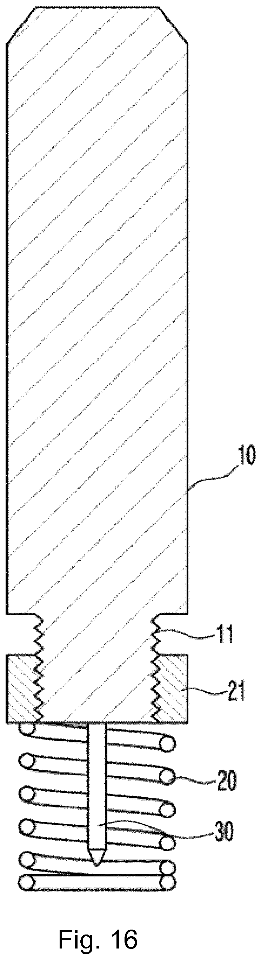

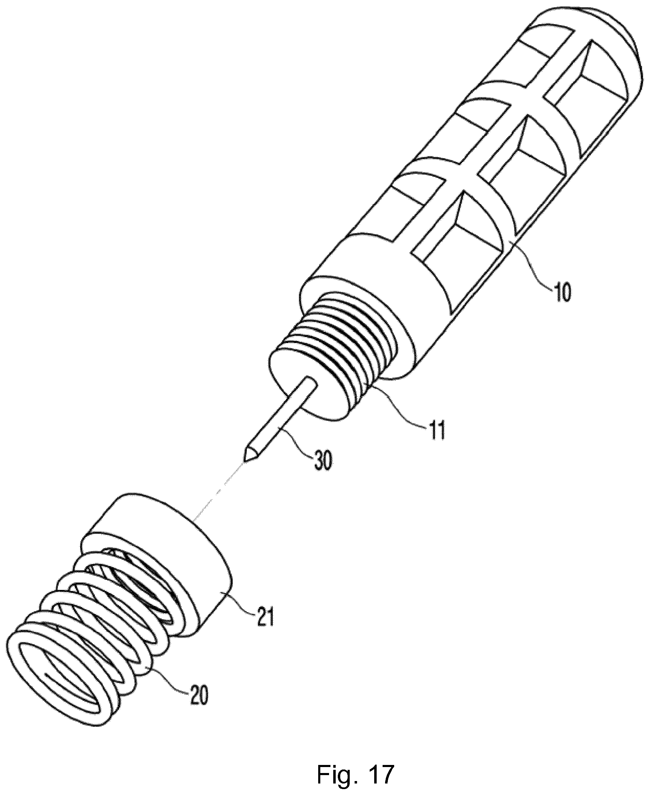

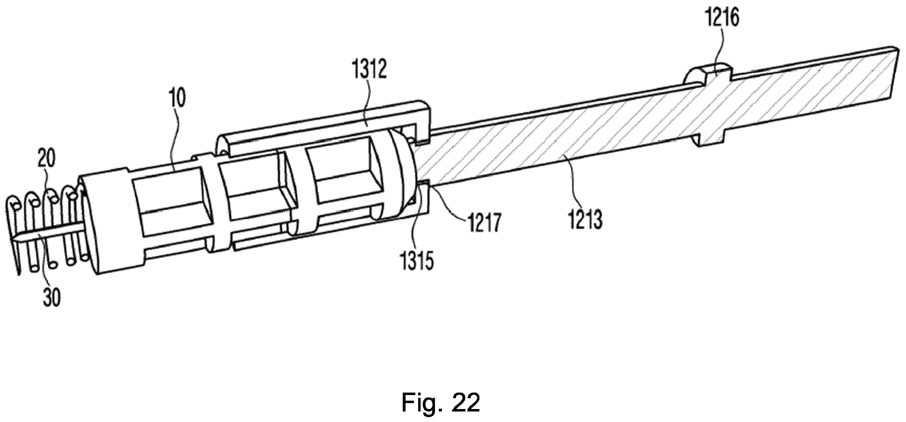

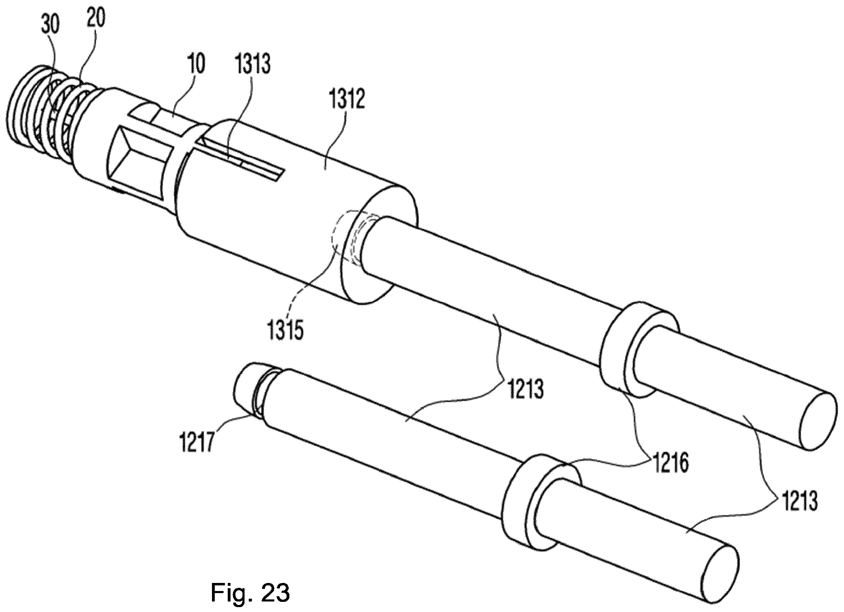

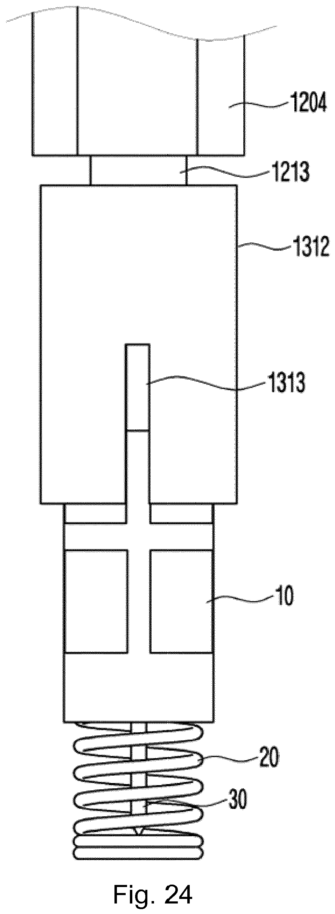

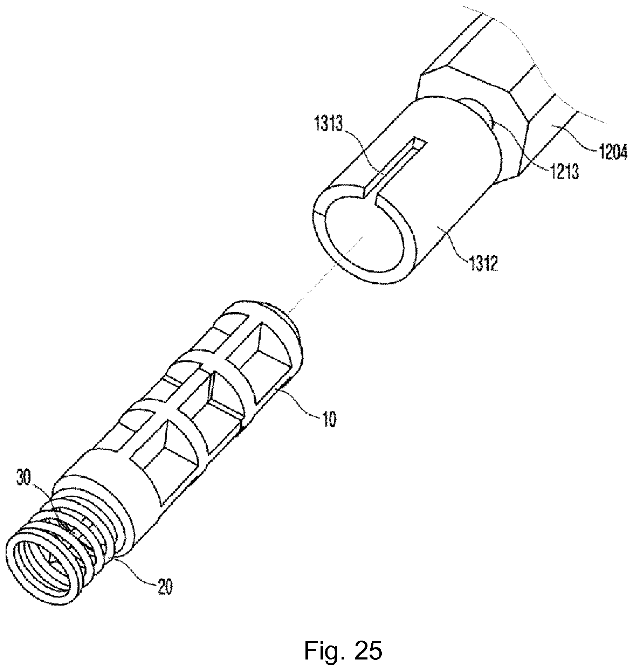

FIGS. **14-26** are drawings of a disposable painless lancet and a lancing device according to a second embodiment of the present invention.

Hereinafter, the disposable painless lancet and the lancing device for therewith according to the second embodiment of the present invention will be described.

As shown in FIG. **14****,** the disposable painless lancet according to the second embodiment of the present invention includes a needle body **10,** a needle **30** embedded in the center of one end of the needle body **10** and a bump spring **20** embedded in one end of the needle body **10** to surround the needle **30.**

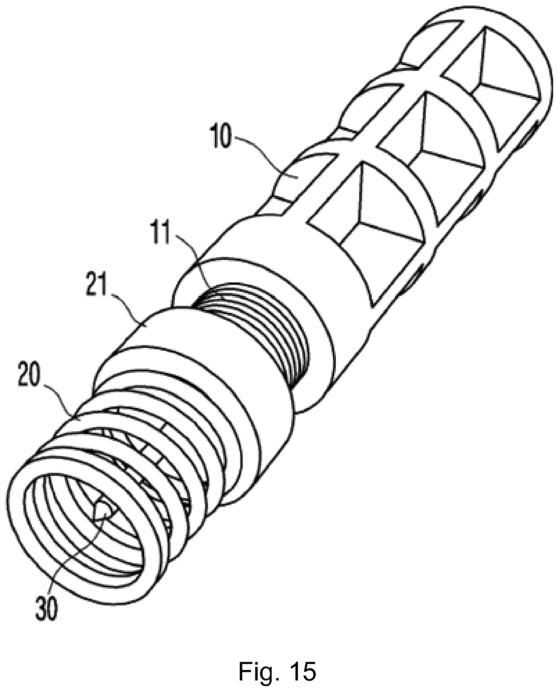

The material of the needle body **10** is preferably plastics. The needle body **10** may have a columnar structure, preferably a skeleton structure as shown in FIGS. **14** and **15****.**

The needle **30** is embedded or fixed at the center of one end of the needle body **10.** As shown in FIG. **14** (a), a part of the bump spring **20** is embedded or fixed to the center of one end of the needle body **10** so as to surround the needle **30** longer than the length of the needle **30.**

The length of the needle **30** can be standardized in the production of the product. In the drawings attached to the present specification, the tip of the needle **30** is conical, but may be angular.

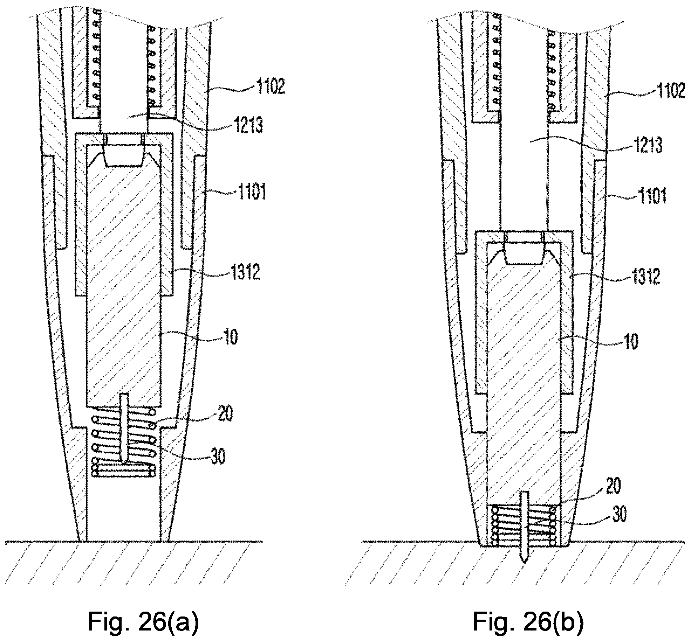

FIG. **14** (b) shows the state of the disposable painless lancet when contacted with the skin (**40** in FIG. **26**) after being launched from the lancing device for sampling blood. When the disposable painless lancet of the present invention is launched from the lancing device, the bump spring **20** momentarily strikes a skin **40.** At this time, the skin **40** is first impacted by the momentary striking applied by the bump spring **20,** disturbing the skin nerve not to feel the pain. After the needle **30** has pierced the skin **40,** the needle **30** is momentarily released out of the skin **40** due to the force of restoration of the bump spring **20** and the time the needle **30** stays on the skin is very short. Also, the needle **30** does not penetrate excessively below the subcutaneous tissue. The bump spring **20** is compressed by inertia at the time of launching and the needle **30** penetrates the skin **40** while pressing the skin **40** harder and harder so that the pain is reduced or eliminated when the needle **30** is pierced. This is like the principle that a nurse in a hospital pierces a needle immediately after striking the skin with the palm of a hand to relieve pain.

As shown in FIG. **26****,** since the bump spring **20** determines a limit of a length that can be pressed immediately after striking the skin **40** and the needle **30** can penetrate the skin **40,** the needle **30** penetrates the skin **40** only by a predetermined length. Therefore, it is possible to prevent the needle **30** from being excessively penetrated into the skin **40** more than necessary for sampling blood.

Also, after the needle **30** has penetrated the skin, the blood sticks to the needle **30** inevitably. However, since the needle **30** is hidden in the bump spring **20,** there is no fear that the blood stuck on the needle **30** comes into contact with a person, thereby preventing secondary infections of various diseases such as AIDS and hepatitis.

FIGS. **15-17** are views showing a disposable painless lancet having a spiral part **11** and a spring body **21,** which are means for adjusting the depth of the needle **30** into the skin **40** according to the second embodiment of the present invention.

One end of the needle body **10** has the columnar spiral part **11** smaller than the diameter of the needle body **10** and one end of the needle **30** is embedded in the center of the end of the spiral part **11.** The spring body **21** is nut-shaped and is screwed onto the spiral part **11.** A spiral is formed inside the spring body **21,** and one end of the bump spring **20** is embedded or fixed to one side of the spring body **21.** When the spring body **21** is turned clockwise, the needle **30** penetrates the skin **40** deeper, and when the spring body **21** is turned counterclockwise, the needle **30** penetrates the skin **40** more shallowly.

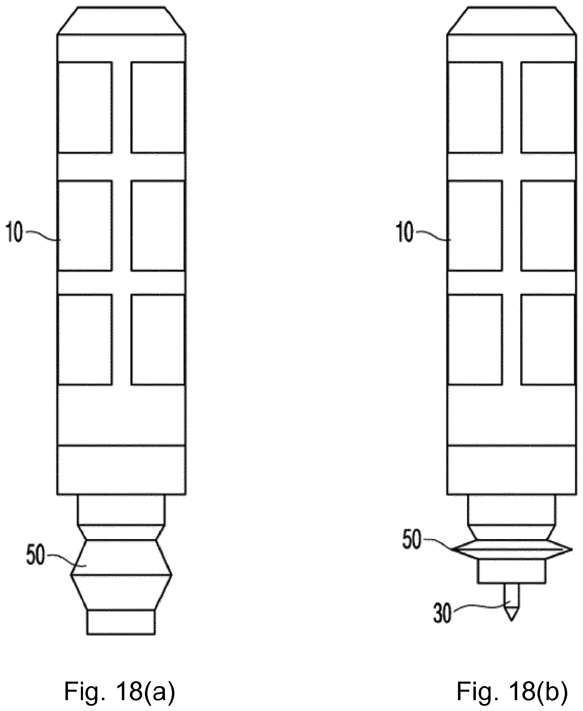

As shown in FIG. **18****,** in the present invention, instead of the bump spring **20,** a silicone or elastic member **50** may be used for a spring function.

In the lancing device according to the second embodiment of the present invention, the principle of operation of the disposable painless lancet in which the silicone or elastic member **50** is used in place of the bump spring **20** is the same as that of the lancet using the bump spring **20.**

Further, the present invention provides a lancing device for use with the disposable painless lancet according to the second embodiment of the present invention.

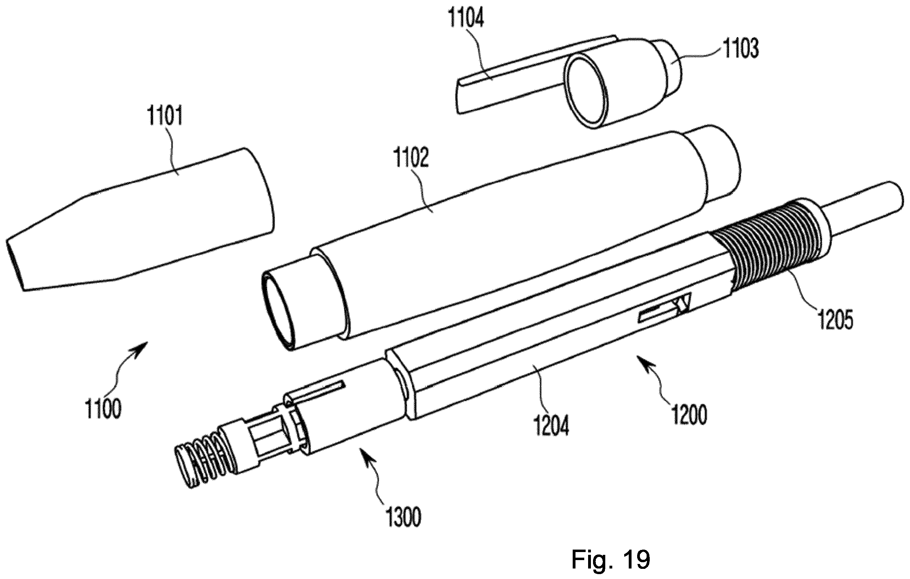

As shown in FIG. **19****,** the lancing device according to the second embodiment of the present invention mainly includes a casing **1100** forming an outer appearance thereof, a launch part **1200** for launching the needle body **10** in a needle body holder **1300,** in which the needle body holder **1300** is configured for holding and fixing the needle body **10.**

As shown in FIG. **19****,** the casing **1100** includes a front cap **1101,** a sleeve **1102** having one end coupled to the front cap **1101** and the other end coupled to the rear cap **1103,** in which the rear cap **1103** is coupled to the sleeve **1102.** The front cap **1101,** the sleeve **1102,** and the rear cap **1103** are preferably screwed together. The rear cap **1103** may be provided with a clip **1104** for convenient carrying.

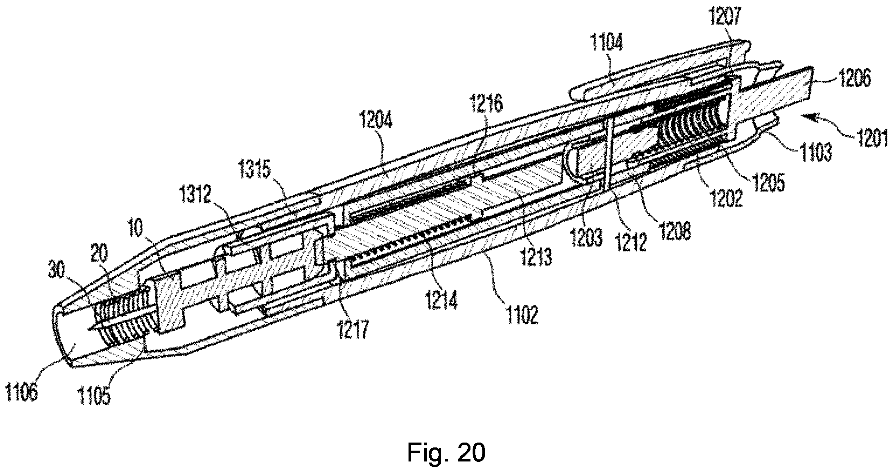

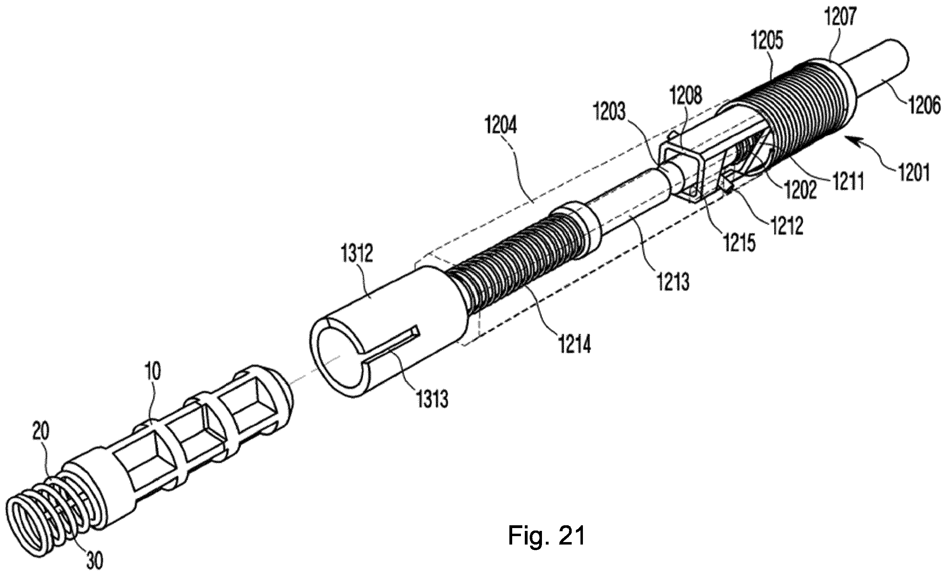

As shown in FIGS. **20** and **21****,** the launch part **1200** includes a launch body **1201,** a launch spring **1202,** a launch pestle **1203,** a square tube **1204,** and a launch body return spring **1205.** As shown in detail in FIGS. **19-21****,** the launch body **1201** integrally comprises a push member **1206,** a launch flange **1207** formed at the tip of the push member **1206,** and a hollow tetrahedron **1208** which is open at one side to accommodate the launch pestle **1203** and the launch spring **1202.** As shown in detail in FIG. **21****,** in the lower part of the hollow tetrahedron **1208,** there are formed square holes **1211** whose upper sides are sloped on one surface and the opposite surface, respectively. The upper sides of the two square holes **1211** are opposite to each other i.e., '\' or '/'). A launch spring **1210** is inserted into one side of the launch pestle **1203** and a trigger pin **1212** is coupled through the middle part of the launch pestle **1203.**

In the lancing device according to the second embodiment of the present invention, the structure and operation principle of the launch part **1200** are as described above with reference to FIGS. **3-7** of the lancing device according to the first embodiment of the present invention.

As shown in detail in FIGS. **20** and **21****,** a ring-shaped protrusion **1315** is formed in the upper position of the needle body holder **1300** so as to be engaged with a coupling groove **1217** of a coupling shaft **1213.** The needle body holder **1300** is open at its lower portion and has one incision groove **1313** cut from the lower portion to the middle portion thereof. The incision groove **1313** is configured to elastically hold the needle body **10** firmly.

As shown in FIG. **20****,** the front cap **1101** of the casing **1100** has a shape gradually tapering from the top to the bottom. A circular engagement protrusion **1105** is formed on the inside of the front cap **1101** to limit the downward movement of the lower surface of a cylindrical body **1312.** A guide hole **1106** is formed from the engagement protrusion **1105** to the lower end of the front cap **1101** to guide the needle body **10** and the bump spring **20** in a predetermined direction.

Referring to FIGS. **20** and **21****,** the operation of the needle body holder **1300** of the lancing device and the disposable painless lancet according to the second embodiment of the present invention will be described below.

When the push member **1206** of the launch part **1200** is pushed to trigger the launch pestle **1203** to strike the coupling shaft **1213,** a force is transmitted to the needle body holder **1310** through the ring-shaped protrusion **1315** formed toward the inner side of the coupling groove **1217** of the coupling shaft **1213** and the cylindrical body **1312** coupled to the coupling groove **1217.** The force is then transmitted to the lancet including the needle body **10** inserted in the cylindrical body **1312,** the bump spring **20** and the needle **30.** Then, the needle body **10** of the lancet and the bump spring **20** are guided through the guide hole **1106** and then the bump spring **20** strikes the skin. The needle **30** penetrates into the capillary blood vessels of the skin while the bump spring **20** is compressed, and then the cylindrical body **1312** of the needle body holder **1300** and the whole lancet are simultaneously returned to the position before the launching by the bump spring **20.**

As described above, since the bump spring **20** strikes the skin to disturb the skin nerves, and then the needle **30** pierces the skin with a slight difference in time, and the time during which the needle **30** stays in the skin **40** is significantly shortened by the restoring force of the bump spring **20,** thereby completely eliminating the pain.

According to the second embodiment of the present invention, when the disposable painless lancet having the silicone or elastic member **50** in place of the bump spring **20** is inserted in the lancing device, the operation principle of the lancing device is the same as the principle described in connection with the bump spring **20.**

The present inventors conducted an experiment for painlessness by use of the lancing device according to the present invention in 50 adult males (mean age 42.5 years). The disposable painless lancets were launched on the skin of their ring fingers. Blood flowed a little, but all 50 people could not feel the pain.

The present invention may also be defined by means of the following numbered clauses.
1. A painless lancing device comprising.
   a casing including:
   a cylindrical front cap;
   a sleeve connected to the cylindrical front cap at a lower end; and
   a rear cap connected to an upper end of the sleeve;
   a launch part disposed in the casing configured to launch a cylindrical bumper and a needle body to strike a skin; and
   a bumper part disposed in the casing and connected to the launch part by a coupling shaft,
   wherein the bumper part comprises a needle body holder including:
   a holder flange disposed at an upper portion thereof;
   a first rectangular hole disposed below the holder flange so that a stop bar moves up and down; and
   a needle body insertion hole disposed at a lower portion of the bumper part to insert a lancet;
   wherein the cylindrical bumper is configured to receive the needle body holder at an upper end thereof, the cylindrical bumper comprising:
   a second rectangular hole disposed along a longitudinal direction to insert and remove the lancet;
   a needle access hole disposed at a lower end of the cylindrical bumper for a needle and a needle spring to move in and out therethrough; and
   a stop bar insertion port disposed at the upper end to insert the stop bar; and
   a bump spring surrounding a cylindrical body and disposed between a lower portion of the holder flange of the needle body holder and an upper portion of the cylindrical bumper.
2. The painless lancing device according to Clause 1, wherein one side of the needle body insertion hole is configured to allow insertion and removal of the needle body from said one side, and the needle body is disposed to align and fit within the needle body insertion hole.
3. The painless lancing device according to Clause 1, wherein the cylindrical body has a lower end configured to allow the needle of the lancet and the needle spring to move in and out,
   wherein a ring-shaped protrusion is disposed at an inside surface of an upper end of the holder flange so that the ring-shaped protrusion is engaged with a circular-shaped coupling groove disposed along a lower outer peripheral surface of the coupling shaft, and
   wherein the holder flange has a screw thread disposed on an outer circumferential surface thereof for screw coupling with a spiral disposed on an inside surface of a depth adjusting member.
4. The painless lancing device according to Clause 1, wherein inner surfaces of the needle body holder have a plurality of needle body fixing protrusions for fixing the needle body.
5. The painless lancing device according to Clause 4, wherein the lancet further comprises:
   a rectangular needle body;
   an upper protrusion and a lower protrusion disposed on an upper side and a lower side of the needle body, respectively, wherein a needle fixing protrusion is disposed on the lower protrusion, the needle spring is inserted and fixed on the needle fixing protrusion, and the needle is inserted into the needle body through the needle fixing protrusion; and
   a plurality of fixing grooves to be fixed on the plurality of needle body fixing protrusions provided on the inner surfaces of the needle body holder.
6. The painless lancing device according to Clause 1, further comprising a depth adjusting member configured to adjust a depth of skin penetration of the needle and having an open upper end and an open lower end, the depth adjusting member comprising:
   a spiral disposed in an inner surface thereof for engaging with a screw thread disposed on an outer circumferential surface of the holder flange of the cylindrical body of the needle body holder; and
   an adjusting member flange disposed at a lower end of the depth adjusting member.
7. The painless lancing device according to Clause 1, wherein the bumper part is connected to the coupling shaft by engaging a ring-shaped protrusion disposed at an inside surface of an upper end of the holder flange with a circular-shaped coupling groove disposed along a lower outer peripheral surface of the coupling shaft, the coupling shaft comprising a coupling shaft flange for inserting a return spring therein.
8. A disposable painless lancet comprising:
   a needle body;
   a needle embedded at a center of one end of the needle body; and
   a bump spring embedded or fixed at said one end of the needle body so as to surround the needle and extend beyond the needle.
9. The disposable painless lancet according to Clause 8,
   wherein the needle body comprises an elongated spiral part disposed at one end thereof; and an end of the needle is embedded in a center of an end of the elongated spiral part,
   wherein a spring body having a screw thread disposed in an inner surface thereof is screw coupled with the elongated spiral part, and
   wherein one end of the bump spring is embedded or fixed to the spring body.
10. The disposable painless lancet according to Clause 8 or 9, comprising a silicone or elastic member instead of the bump spring.
11. A painless lancing device, comprising:
   a casing including:
      a cylindrical front cap;
      a sleeve connected to the cylindrical front cap at a bottom end; and
      a rear cap connected to a top end of the sleeve;
   a launch part for launching a needle body disposed inside a cylindrical body; and
   a needle body holder for holding and fixing the needle body ,
   wherein the cylindrical front cap has a gradually tapering shape from a top end to a bottom end, and the cylindrical front cap comprises:
      a circular engagement protrusion disposed on an inside surface of the cylindrical front cap to limit a downward movement of a bottom surface of the cylindrical body; and
      a guide hole provided from the circular engagement protrusion to the bottom end of the cylindrical front cap to guide the needle body and a bump spring in a predetermined direction,
   wherein the needle body holder comprises a ring-shaped protrusion disposed in an inside surface of the needle body holder so as to be engaged with a coupling groove of a coupling shaft, and
   wherein the needle body holder is open at a bottom portion and has one incision groove on its side provided from the lower portion to a middle portion thereof.

## Claims

1. A disposable painless lancet comprising:
a needle body **10;**
a needle **30** embedded at a center of one end of the needle body **10;** and
a bump spring **20** embedded or fixed at said one end of the needle body **10** so as to surround the needle **30** and extend beyond the needle **30.**

2. The disposable painless lancet according to Claim 1,
wherein the needle body **10** comprises an elongated spiral part **11** disposed at one end thereof; and an end of the needle **30** is embedded in a center of an end of the elongated spiral part **11,**
wherein a spring body **21** having a screw thread disposed in an inner surface thereof is screw coupled with the elongated spiral part **11,** and
wherein one end of the bump spring **20** is embedded or fixed to the spring body **21.**

3. The disposable painless lancet according to Claim 1 or 2, comprising a silicone or elastic member **50** instead of the bump spring **20.**
